# EUROPEAN PATENT APPLICATION

(11) **EP 4 154 746 A1**
(43) Date of publication of application: **29.03.2023**
(21) Application number: 21809166.8
(22) Date of filing: 06.05.2021
(51) Int. Cl.: A41D 13/12, A61B 5/256, A61B 5/263

(54) **CLOTHING-TYPE BIOMETRIC DATA MEASUREMENT DEVICE AND MANUFACTURING METHOD THEREOF**

(30) Priority: 20.05.2020 JP 2020088254
(71) Applicant: TOYOBO CO., LTD., Osaka-shi Osaka 5300001 (JP)
(72) Inventor: IRIE, Michihiko, Otsu-shi, Shiga 520-0292 (JP); SHIMIZU, Yusuke, Otsu-shi, Shiga 520-0292 (JP); OMOTE, Yuichiro, Osaka-shi, Osaka 530-8230 (JP); MAEDA, Satoshi, Otsu-shi, Shiga 520-0292 (JP)
(74) Representative: Müller-Boré & Partner Patentanwälte PartG mbB
(86) International application number: PCT/JP2021/017408
(87) International publication number: WO 2021/235235

(57) **Abstract**

An object is to provide a garment-type biological information measurement device having a biological contact-type electrode and having excellent durability against washing.

A garment-type biological information measurement device comprising an electrode, wherein the electrode comprises a stretchable conductive layer containing a flexible resin and a conductive filler, a stretchable adhesion acceleration layer, a stretchable hot melt adhesive layer, and a garment fabric, in this order.

## Description

### TECHNICAL FIELD

The present invention relates to a garment-type biological information measurement device using an electrode containing a stretchable conductive layer.

### BACKGROUND ART

A garment-type biological information measurement device for measuring biological information such as electrocardiographic information has been developed. In such a garment-type device, a cloth fabric forming the garment needs to be equipped with an electrode or electrical wiring. The electrode and the electrical wiring each have to be made from a material that is deformable in a stretching manner according to deformation of the garment. As technologies related to such a deformable electrode and such deformable electrical wiring, technologies have been known in which a conductor such as electrical wiring made from a conductive yarn or metal foil made into a meandering pattern is disposed so as to have a degree of freedom for deformation thereof so that the conductor can follow deformation of a garment fabric.

For example, Patent Document 1 discloses a knitted fabric that is made from a conductive yarn and that can be used as a to-be-worn material for a wearable computer or the like to be worn on a human body.

In addition, attempts regarding a technology in which wiring is formed on a fabric by using a conductive printing ink have been made. For example, Patent Document 2 discloses an electronic fabric or an electronic garment made from the electronic fabric, each including: an electronic device such as a luminescent device or a sensor; and a wiring pattern, an electronic circuit, and the like that cause the electronic device to function. In the electronic fabric or the electronic garment, any or all of the electronic device, the wiring pattern, the electronic circuit, and the like are formed on a fabric surface by using a conductive ink or the like through a printing method.

Further, a technology in which a stretchable conductive composition obtained from an elastomer and a conductive filler is used as an electrode or wiring, has been proposed. For example, Patent Document 3 discloses a method for manufacturing a stretchable electrode sheet. The method includes: a step of applying a conductive paste containing a stretchable resin onto a release sheet and drying the conductive paste, to make a stretchable conductor layer; a step of superposing a first hot melt sheet onto the stretchable conductor layer, to obtain a three-layer sheet; a step of punching a portion of the three-layer sheet; and a step of superposing the three-layer sheet after the step of punching onto a fabric such that the first hot melt sheet and the fabric are in contact with each other, and performing hot-pressing.

In addition, Patent Document 4 discloses a stretchable conductive film for textiles. The stretchable conductive film for textiles includes: a stretchable conductive layer having stretchability; and a hot melt adhesive layer formed on one surface of the stretchable conductive layer. The stretchable conductive layer is made from a conductive composition containing: an elastomer; and a conductive filler with which the elastomer is filled.

It can be understood that these technologies are technologies for making garments into electronics. However, for garments, there are various situations that are different from those for conventional electronics products in terms of use cases. A particularly problematic situation is one involving an operation of washing that is inevitable for garments. Generally speaking, electronics products are designed and manufactured without assuming a situation in which the electronics products are thrown into a washing machine.

A conductive yarn is made mainly by a method such as one involving plating the surface of a non-conductive yarn with a metal. There is a case where the metal with which the plating has been performed peels from such a conductive yarn owing to intense friction during washing so that the conductivity thereof decreases.

The same applies to the case of using a conductive printing ink. That is, there is a case where problems such as peeling of the ink owing to friction, repetitive bending, compression, or the like during washing occur.

A stretchable conductive layer obtained from a stretchable conductive composition is relatively highly resistant to repetitive bending, compression, and friction. Thus, if the stretchable conductive layer can be adhered on a fabric of a garment by appropriate means, a high durability against washing can be expected.

A hot melt adhesive sheet is of a solventless type, and thus is easy to handle. Further, a hot melt adhesive sheet can be used without any problem even at manufacturing sites in the apparel industry, in which ventilation equipment such as one in chemical plants is not provided. Thus, at manufacturing sites for garments, a hot melt adhesive sheet is widely used for adhesion of interlining fabrics and adhesion of garment parts. Therefore, it can be said that a hot melt adhesive sheet is one of means suitably applicable also to the case where a stretchable conductive layer is adhered on a garment.

### RELATED ART DOCUMENTS

### PATENT DOCUMENTS

Patent Document 1: JP-A-2007-191811
Patent Document 2: JP-A-2005-146499
Patent Document 3: JP-A-2018-102965
Patent Document 4: JP-A-2017-101124

### DISCLOSURE OF THE INVENTION

### PROBLEMS TO BE SOLVED BY THE INVENTION

However, a hot melt adhesive sheet manufactured in consideration of adhesion to a garment fabric is not always preferable in terms of adhesiveness to a material other than garment fabrics. In general, a stretchable conductive composition contains a conductive filler and a flexible binder resin as main components. Conductivity is imparted by direct contact between conductive fillers. Thus, the blending amount of the binder resin is much smaller than that of a generally used coating, and the binder resin is blended such that the amount thereof is smaller than an amount for achieving complete filling between the conductive fillers in a three-dimensional geometric manner. As a result, a portion at which the conductive fillers are directly exposed is present on a surface of the resultant conductive layer. As a matter of course, this presence is advantageous in the case of using the conductive layer as an electrode. However, if adhesion of the conductive layer to another material by using an adhesive is considered, the adhesive is required to have adhesiveness to both the binder resin and the conductive fillers. In many cases, conductive fillers are metal powders which are mainly silver powder. Hot melt adhesive sheets used for apparel are not always designed in consideration of adhesiveness to the surfaces of these metal powders.

As expected, lack of the adhesiveness often becomes prominent during washing. Specifically, the stretchable conductive layer adhered by using a hot melt adhesive sheet might peel at the interface between the conductive layer and the hot melt adhesive sheet when washing is performed repetitively. Since the mechanical strength of the conductive layer is not higher than that of a nearby material, the portion having peeled falls off from the garment. This often leads to the case where a wiring function of the garment is irreparably impaired.

That is, although the technology in which a stretchable conductive layer is adhered on a garment fabric by using a hot melt adhesive sheet is easy to use when electrical wiring is provided to the garment, there has been room for improvement in durability against washing, which is a problem specific to garments.

The present inventors conducted thorough research for solving this problem. As a result, the present inventors have solved this problem by providing an adhesion acceleration layer interposed between a stretchable conductive layer and a hot melt adhesive layer, thereby arriving at realization of a garment-type biological information measurement device having high durability against washing.

### SOLUTIONS TO THE PROBLEMS

That is, the present invention consists of the following configurations.
[1] A garment-type biological information measurement device comprising
   an electrode (hereinafter, also referred to as an electrode portion), wherein the electrode comprises
   a stretchable conductive layer containing a flexible resin and a conductive filler,
   a stretchable adhesion acceleration layer,
   a stretchable hot melt adhesive layer, and
   a garment fabric, in this order.
[2] The garment-type biological information measurement device according to [1], wherein
   the device further comprises a stretchable surface insulation layer disposed on a side, of the conductive layer, that is brought into contact with a living body.
[3] The garment-type biological information measurement device according to [1] or [2], wherein
   the conductive layer has a structure with two or more layers including
   a stretchable first conductive layer containing at least a flexible resin and a carbon-based conductive filler as constituent components, and
   a stretchable second conductive layer containing at least a flexible resin and a metal-based conductive filler as constituent components.
[4] A manufacturing method for the garment-type biological information measurement device according to [1] or [2],
   the manufacturing method comprising:
   making, on a temporary support body, a to-be-pasted-on-garment-fabric conductive laminated sheet through a process comprising at least
      a step of forming a stretchable conductive layer containing a flexible resin and a conductive filler,
      a step of forming a stretchable adhesion acceleration layer on the conductive layer, and
      a step of forming a stretchable hot melt adhesive layer on the adhesion acceleration layer; and
   pasting the obtained to-be-pasted-on-garment-fabric conductive laminated sheet on a garment via the hot melt adhesive layer, to form an electrode on the garment.
[5] A manufacturing method for the garment-type biological information measurement device according to [3],
   the manufacturing method comprising:
   making, on a temporary support body, a to-be-pasted-on-garment-fabric conductive laminated sheet through a process comprising at least
      a step of forming a stretchable first conductive layer containing at least a flexible resin and a carbon-based conductive filler as constituent components,
      a step of further forming a stretchable second conductive layer containing at least a flexible resin and a carbon-based conductive filler as constituent components,
      a step of forming a stretchable adhesion acceleration layer on the second conductive layer, and
      a step of forming a stretchable hot melt adhesive layer on the adhesion acceleration layer; and
   pasting the obtained to-be-pasted-on-garment-fabric conductive laminated sheet on a garment via the hot melt adhesive layer, to form an electrode on the garment.
[6] The garment-type biological information measurement device according to any one of [1] to [3], and the manufacturing method, for the garment-type biological information measurement device according to [4] or [5], wherein
   the adhesion acceleration layer contains an elastomer having a crosslinked structure.
[7] The garment-type biological information measurement device according to any one of [1] to [3], and the manufacturing method for the garment-type biological information measurement device according to [4] or [5], wherein
   the adhesion acceleration layer contains a urethane resin having a crosslinked structure.
[8] The garment-type biological information measurement device according to any one of [1] to [3], and the manufacturing method for the garment-type biological information measurement device according to [4] or [5], wherein
   the adhesion acceleration layer contains an active energy ray-curable acrylic resin composition.

Further, the present invention preferably has the following configurations.
[9] A garment-type biological information measurement device comprising
   an electrode that contacts with the surface of a living body, wherein
   the electrode has a layered structure comprising
      a stretchable conductive layer composed of at least a flexible resin and a conductive filler,
      a stretchable adhesion acceleration layer,
      a stretchable first hot melt adhesive layer,
      a stretchable insulation layer,
      a stretchable second hot melt adhesive layer, and a garment fabric, in this order.
[10] A garment-type biological information measurement device comprising
   electrical wiring, wherein
   the electrical wiring has a layered structure comprising
      a stretchable conductive layer composed of at least a flexible resin and a conductive filler,
      a stretchable adhesion acceleration layer,
      a stretchable first hot melt adhesive layer,
      a stretchable insulation layer,
      a stretchable second hot melt adhesive layer, and a garment fabric, in this order.
[11] A garment-type biological information measurement device comprising
   an electrode that contacts with the surface of a living body, wherein
   the electrode has a layered structure comprising
      a stretchable first conductive layer composed of at least a flexible resin and a carbon-based conductive filler,
      a stretchable second conductive layer composed of at least a flexible resin and a metal-based conductive filler,
      a stretchable adhesion acceleration layer,
      a stretchable first hot melt adhesive layer,
      a stretchable insulation layer,
      a stretchable second hot melt adhesive layer, and
      a garment fabric, in this order.
[12] A garment-type biological information measurement device comprising
   electrical wiring, wherein
   the electrical wiring has a layered structure comprising
      a stretchable first conductive layer composed of at least a flexible resin and a carbon-based conductive filler,
      a stretchable second conductive layer composed of at least a flexible resin and a metal-based conductive filler,
      a stretchable adhesion acceleration layer,
      a stretchable first hot melt adhesive layer,
      a stretchable insulation layer,
      a stretchable second hot melt adhesive layer, and
      a garment fabric, in this order.

### EFFECTS OF THE INVENTION

In the present invention, because the adhesion acceleration layer is inserted between the stretchable conductive layer and the hot melt adhesive layer, the adhesiveness between the conductive layer and the hot melt adhesive layer is stabilized. The reason for this is considered to be because the adhesion acceleration layer has high adhesiveness to both the surface of the conductive filler (particularly, metallic filler) of the conductive layer and the hot melt adhesive layer.

Firstly, each surface of the stretchable conductive layer is not a smooth surface in a microscopic view. When the surface is observed in a magnified view, bumps of the conductive filler (particularly, metallic filler) are exposed like rocks, and the gaps between the bumps are filled with the flexible resin. That is, this situation is likened to a sight of a rock garden. When adhesion to such a significantly rough surface is performed by using the hot melt adhesive, heating needs to be performed so as to sufficiently soften the hot melt adhesive. In actuality, however, it is difficult to increase the temperature to a temperature for the sufficient softening owing to limitations imposed by, for example, the heat resistances of a nearby material and the conductive layer itself adhesion to which is to be performed. Even in the case of using a hot melt adhesive having a low softening temperature, the hot melt adhesive has a lower ability to follow a rough surface than an adhesive, the initial form of which is liquid form, such as a so-called solvent adhesive. Thus, it is difficult to completely join together adhesion surfaces by using this hot melt adhesive. In addition, in the case where the softening temperature has been decreased more than necessary, a problem is likely to occur in the case of subjection to a relatively high temperature as in: an operation such as ironing; a drying step after washing; or the like.

As a result, in the case of using the hot melt adhesive, the area in which adhesion is actually achieved is smaller than a nominal adhesion area.

Macroscopically, a hot melt adhesive resin used for apparel is designed to be relatively flexible in terms of mechanical properties thereof so as to be able to follow deformation of a garment fabric to some extent. Meanwhile, a hot melt adhesive intended to be used for apparel is not always designed in consideration of adhesion to a metal.

When such a hot melt adhesive is used for adhesion to the conductive layer, the hot melt adhesive is strongly adhered only to the resin portion that is present on the surface of the conductive layer and has a weak adhesiveness to the conductive filler (particularly, metallic filler). When deformation stress is applied to such an adhesion interface, a portion adhered on the conductive filler peels first, and the stress is further concentrated on the adhesion interface between the hot melt adhesive and the resin portion.

If contact surfaces of the hot melt adhesive and the conductive filler are separated from each other, a minute air gap is generated therebetween. In an air atmosphere, the inside of the air gap is filled with air which is a compressible fluid. However, during washing, a detergent solution, i.e., an aqueous fluid containing a surfactant, enters the air gap. Since an aqueous fluid is incompressible, the liquid held in the minute space at this interface attempts to move to another location owing to a pressure applied by deformation such as stretching, bending, compression, or twisting in association with a washing operation, and is pressed in the direction of separation between the adhesion surfaces of the hot melt adhesive and the resin portion adj acent to each other. Alternatively, the liquid attempts to move to a free space inside the conductive layer which is originally inhomogeneous. The detergent solution has a low surface tension and has excellent wetting ability with respect to a solid surface, and thus easily enters a narrow gap and a small air gap. As a result, the detergent solution acts so as to cause peeling at the interface between the conductive layer and the hot melt adhesive layer. Furthermore, the detergent solution acts so as to weaken the conductive layer itself from inside so that the conductive layer decomposes owing to a load applied by washing.

As a result of these actions, use of a conventional hot melt adhesive to adhere the conductive layer to a garment fabric causes a portion of the conductive layer to decompose and fall off when washing is repetitively performed.

The adhesion acceleration layer in the present invention is preferably used in liquid form. Thus, the adhesion acceleration layer is in close contact with both the resin portion and the conductive filler portion in the intensely rough surface of the conductive layer and exhibits high adhesiveness. In addition, since the adhesion acceleration layer has smooth surfaces, the adhesion acceleration layer exhibits favorable adhesiveness also to the hot melt adhesive layer.

Therefore, even if washing is repetitively performed, a favorable adhesion state can be maintained without causing microscopic peeling due to local concentration of stress or decomposition of the conductive layer due to a detergent solution having entered a microscopic peel interface which have been described above.

### BRIEF DESCRIPTION OF THE DRAWINGS

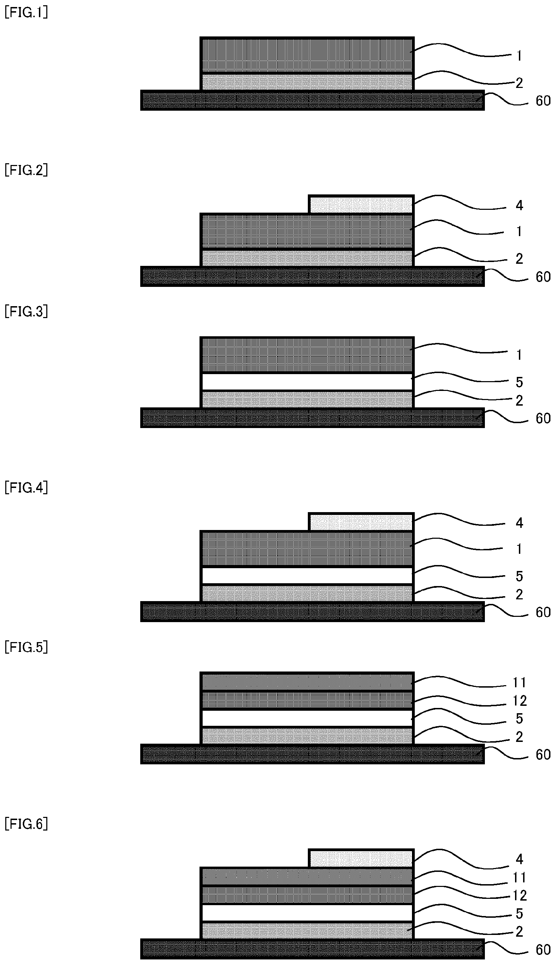

FIG. 1 is a schematic diagram showing a cross-sectional structure of an electrode portion of a conventional garment-type biological information measuring device.
FIG. 2 is a schematic diagram showing a cross-sectional structure of an electrode portion of a conventional garment-type biological information measuring device in which a non-thermoplastic film corresponding to a cover insulation layer partially covers a conductive layer.
FIG. 3 is a schematic diagram showing the cross-sectional structure of the electrode portion of a garment-type biological information measuring device of the present invention.
FIG. 4 is a schematic diagram showing a cross-sectional structure of an electrode portion of a garment-type biological information measuring device of the present invention in which a non-thermoplastic film corresponding to a cover insulation layer partially covers a conductive layer. A portion having a structure covered with a cover insulation layer can be regarded as an electric wiring portion (wiring) that is insulated and protected. The same applies to the following examples.
FIG. 5 is a schematic diagram showing a cross-sectional structure in the case where the stretchable conductive layer in the electrode portion of a garment-type biological information measuring device of the present invention has a two-layer structure of a first conductive layer and a second conductive layer.
FIG. 6 is a schematic diagram showing a cross-sectional structure, which shows a case where the stretchable conductive layer in the electrode portion of a garment-type biological information measuring device of the present invention has a two-layer structure of a first conductive layer and a second conductive layer, and further a non-thermoplastic film corresponding to a cover insulation layer partially covers the conductive layer.
FIG. 7 is a schematic process diagram showing an example of a process for actually manufacturing an electrode portion and an electrical wiring portion of a garment-type biological information measuring device of the present invention.
FIG. 8 is an example of a pattern diagram having electrodes and wiring for performing electrocardiographic measurement, and a connection portion with a connector.

### MODE FOR CARRYING OUT THE INVENTION

In the present invention, a to-be-pasted-on-garment-fabric conductive laminated sheet including at least a stretchable conductive layer, an adhesion acceleration layer, and a hot melt adhesive layer in this order and further including a surface insulation layer, a release film, and the like as necessary, is produced, and the conductive laminated sheet is pasted on a garment fabric by using the hot melt adhesive layer, whereby a garment-type biological information measurement device is obtained.

The stretchable conductive layer in the present invention refers to a layer containing at least a flexible resin and a conductive filler as constituent components.

The stretchable conductive layer preferably refers to a stretchable layer made from a material having a specific resistance of not higher than 1×10¹ Ωcm. The stretchable conductive layer in the present invention has stretchability. The stretchability in the present invention refers to the ability to be repetitively stretched by at least 10% in a state where conductivity is maintained. Further, the stretchable conductive layer in the present invention preferably has an elongation at break of not lower than 40% alone, more preferably has an elongation at break of not lower than 50% alone, and further preferably has an elongation at break of not lower than 80% alone. Further, the stretchable conductive layer in the present invention preferably has a tensile elastic modulus of 0.5 to 300 MPa. A material from which such a stretchable conductive layer having stretchability can be formed is referred to as a stretchable conductor composition. The stretchable conductor composition is a complex containing at least the flexible resin and the conductive filler.

In the present invention, the flexible resin includes thermoplastic resins, thermosetting resins, rubbers and the like, which preferably have an elastic modulus of 0.1 to 1000 MPa. The flexible resin is preferably thermoplastic resins or rubbers, and is more preferably urethane resin or rubbers, in order to develop the film stretchability.

Examples of the rubbers include urethane rubber, acrylic rubber, silicone rubber, butadiene rubber, rubber containing a nitrile group such as nitrile rubber or hydrogenated nitrile rubber, isoprene rubber, vulcanized rubber, styrene-butadiene rubber, butyl rubber, chlorosulfonated polyethylene rubber, ethylene propylene rubber, vinylidene fluoride copolymer, and the like. Among these, rubber containing a nitrile group, chloroprene rubber, and chlorosulfonated polyethylene rubber are preferable, and rubber containing a nitrile group is particularly preferable. The elastic modulus in the present invention is preferably within a range of 0.1 to 600 MPa, more preferably 0.2 to 500 MPa, further preferably 0.5 to 300 MPa.

There is no particular limitation for the rubber containing a nitrile group as far as it is a rubber or elastomer containing a nitrile group, and nitrile rubber and hydrogenated nitrile rubber are preferable.
Nitrile rubber is a copolymer of butadiene with acrylonitrile, and when the amount of bonding acrylonitrile increases, affinity with metal increases but rubber elasticity contributing to stretchability rather decreases. Therefore, the amount of bonding acrylonitrile in the acrylonitrile butadiene copolymer rubber is preferably 18 to 50% by mass, and more preferably 40 to 50% by mass.

The urethane resin is preferably a polyurethane elastomer. The polyurethane elastomer has a glass transition temperature (Tg) that is preferably not lower than -60°C and more preferably not lower than -50°C. The upper limit of the glass transition temperature is preferably 10°C and more preferably 0°C.

The polyurethane elastomer in the present invention is preferably a polyurethane elastomer obtained by reacting: a soft segment made from a polyether-based polyol, a polyester-based polyol, a polycarbonate-based polyol, or the like; and a hard segment made from diisocyanate or the like. The soft segment component is more preferably a polyester polyol in terms of the degree of freedom in molecule design.

Examples of the polyether-based polyol in the present invention include polyethylene glycol, polypropylene glycol, polypropylene triol, polypropylene tetraol, polytetramethylene glycol, polytetramethylene triol, polyalkylene glycols such as copolymers obtained by copolymerizing monomer materials such as cyclic ethers for synthesizing these polyether-based polyols, derivatives and modified products obtained by introducing side chains or branch structures to these polyether-based polyols, mixtures of these polyether-based polyols, and the like. Among these polyether-based polyols, polytetramethylene glycol is preferable. The reason for this is because mechanical properties thereof are excellent.

As the polyester-based polyol in the present invention, it is possible to use an aromatic polyester polyol, an aromatic/aliphatic copolymerized polyester polyol, an aliphatic polyester polyol, or an alicyclic polyester polyol. As the polyester polyol in the present invention, a saturated or unsaturated polyester polyol may be used. Among these polyester polyols, an aliphatic polyester polyol is preferable.

Conductive filler in the present invention is composed of a material having a specific resistance of 1×10⁻¹Ωcm or less. And conductive filler has a particle diameter of 100 µm or less. Examples of the material having a specific resistance of 1×10⁻¹Ωcm or less include metal, alloy, carbon, doped semiconductor, conductive polymer, and the like. As the conductive filler preferably used in the present invention, metals such as silver, gold, platinum, palladium, copper, nickel, aluminum, zinc, lead, and tin, alloy particles such as brass, bronze, cupronickel, and solder, hybrid particles such as silver-coated copper, metal-plated polymer particles, metal-plated glass particles, metal-coated ceramic particles, and the like can be used. These are called a metal-based conductive filler.

In the present invention, it is preferred to use flaky silver particles or an irregular-shaped aggregated silver powder as a metal-based conductive filler. Although there is no particular limitation for the particle diameter of the flaky powder, the average particle diameter (50% D) measured by a dynamic light scattering method is preferably 0.5 to 20 µm, and more preferably 3 to 12 µm. If the average particle diameter exceeds 15 µm, the formation of a fine wiring may become difficult, and clogging occurs in the case of screen printing or the like. If the average particle diameter is less than 0.5 µm, the particles cannot contact with each other when the filling amount is small, and as a result, the electrical conductivity may deteriorate. Although there is no particular limitation for the particle diameter of the irregular-shaped aggregated powder, the average particle diameter (50% D) measured by a light scattering method is preferably 1 to 20 µm, and more preferably 3 to 12 µm. If the average particle diameter exceeds 20 µm, the dispersibility decrease, and as a result, paste formation may become difficult. If the average particle diameter is less than 1 µm, the effects as the aggregated powder is lost, and as a result, high electrical conductivity may not be maintained when the filling amount is small.

In the present invention, a carbon-based conductive filler can be used.

As the carbon-based conductive filler in the present invention, graphite powder, activated carbon powder, flake graphite powder, acetylene black, Ketjenblack, fullerene, single-walled carbon nanotube, multi-walled carbon nanotube, carbon nanocone and the like can be used. In the present invention, graphite powder, flake graphite powder, activated carbon powder, and Ketjenblack are preferably used as the carbon-based conductive filler. In the present invention, it is further preferable to use carbon-based conductive filler at least having a BET specific surface area of 1000 m²/g or more.

The stretchable conductor composition for forming the stretchable conductive layer in the present invention contains at least the conductive filler and the flexible resin (hereinafter, also referred to as a binder). In the case of a metal-based conductive filler, the metal-based conductive filler can be blended in an amount of 40 to 92% by mass relative to the total mass of the metal-based conductive filler and the binder. The metal-based conductive filler in the present invention is preferably contained in an amount of 50 to 90% by mass, more preferably contained in an amount of 58 to 89% by mass, further preferably contained in an amount of 66 to 88% by mass, and even more preferably contained in an amount of 70 to 87% by mass, relative to the total mass of the metal-based conductive filler and the binder. When the metal-based conductive filler content is set to fall within this range, the conductive layer can obtain a sufficient elongation for exhibiting required conductivity and stretchability.

In the case of using a carbon-based conductive filler as the conductive filler, the carbon-based conductive filler can be blended in an amount of 18 to 65% by mass relative to the total mass of the carbon-based conductive filler and the binder. The carbon-based conductive filler in the present invention is preferably contained in an amount of 22.5% by mass or more, more preferably 25% by mass or more, further preferably 27.5% by mass or more, and still further preferably 30% by mass or more relative to the total mass of the carbon-based conductive filler and the binder. In the case of a carbon-based conductive filler, the carbon-based conductive filler can be blended in an amount of 62.5% by mass or less, more preferably 60% by mass or less relative to the total mass of the carbon-based conductive filler and the binder. When the carbon-based conductive filler content is set to fall within predetermined range, the conductive layer can obtain a sufficient elongation for exhibiting required conductivity and stretchability.

In the present invention, non-conductive particles may be blended in the stretchable conductor composition. The non-conductive filler in the present invention is preferably particles as an organic or inorganic insulating substance. The inorganic particles in the present invention are added for the purpose of improving printing properties, stretching properties, and coating film surface properties. As the inorganic particles, it is possible to use: silica, titanium oxide, talc, or alumina which are metal oxides; glass beads; a phosphate/carbonate, a titanate, a carbonate, or a sulfate of an alkali metal or an alkali earth metal, e.g., barium sulfate, sodium phosphate, or the like; a mixture of these inorganic particles; inorganic particles of, for example, kaolin derived from a natural substance; a microgel made from a resin material; or the like.

The stretchable conductor composition can be produced by blending the flexible resin, the conductive filler, and other necessary components and melt-kneading the mixture by using an extruder or the like. Alternatively, the stretchable conductor composition can be obtained also by, after adding a solvent and kneading the mixture in the form of a slurry, applying the slurry onto a certain base material and drying the slurry so as to remove the solvent. In consideration of performing layer thinning in order to obtain the stretchable conductive layer, the latter method in which a solvent is used and the mixture is kneaded in the form of a slurry is preferable. The mixture in the form of a slurry containing the conductive filler, the flexible resin, and the solvent can also be used as an ink or a paste in a printing step. In this case, additives such as a defoamer, a leveling agent, and a thixotropy imparting agent may further be added.

In one aspect of the present invention, a combination can be presented as an example. In the combination, a stretchable conductive layer made from the flexible resin and the carbon-based conductive filler is used as a stretchable first conductive layer, and a stretchable conductive layer made from the flexible resin and the metal-based conductive filler is used as a stretchable second conductive layer. In this case, the first conductive layer can be used as an electrode layer to be brought into contact with a living body.

A contaminant from the environment or a body fluid of a biological origin such as sweat, tear, or saliva, is adhered on an electrode surface that is directly in contact with the surface of the living body. Thus, the surface of the metal-based filler is oxidized or sulfurized, and the contact resistance thereof is increased. Consequently, in some cases, the electrode surface might be insulated. However, although the carbon-based conductive filler has a lower conductivity than the metal filler, the carbon-based conductive filler has a high chemical resistance, and thus can be preferably used as a conductive filler for a conductive layer that is formed as an electrode surface layer.

In the present invention, a stretchable surface insulation layer can be provided on the stretchable conductive layer. Specifically, the surface of a portion, of the conductive layer, that is not required to function as a living body contact electrode and that is used for merely transmitting a signal or power, can be insulated from the viewpoint of protecting the conductive layer, preventing noise mixing, or safety on the living body side.

As the stretchable surface insulation layer, it is possible to use a resin material equivalent to the flexible resin used as a conductive binder resin. It is more preferable to use a resin in which a crosslinked structure has been moderately provided by using a conductive binder resin as a basic structure.

Specifically, the stretchable surface insulation layer is preferably urethane rubber, acrylic rubber, silicone rubber, butadiene rubber, rubber containing a nitrile group such as nitrile rubber or hydrogenated nitrile rubber, isoprene rubber, vulcanized rubber, styrene-butadiene rubber, butyl rubber, chlorosulfonated polyethylene rubber, ethylene propylene rubber, vinylidene fluoride copolymer or the like, and further, a rubber material obtained by introducing a crosslinked structure into any of these rubber materials is preferably used. As the urethane resin, it is possible to use a polyurethane elastomer obtained by combining: a soft segment made from a polyether-based polyol, a polyester-based polyol, a polycarbonate-based polyol, or the like; and a hard segment made from diisocyanate or the like. By blending a trifunctional or higher-functional polyol in the polyurethane elastomer, a crosslinked structure can easily be formed.

In the present invention, a stretchable hot melt adhesive layer is provided. The hot melt adhesive layer allows the conductive laminated sheet for attaching to a garment fabric of the present invention to be adhered to stretchable objects such as a garment fabric.

The hot melt adhesive layer in the present invention is preferably an adhesive composed of a thermoplastic resin capable of adhering to a fabric by heating at a temperature in the range of 45° C. to 250° C., or a thermosetting resin in an uncured or half-cured state, which is called a reactive hot melt adhesive. More specifically, a polyester-based, polyurethane-based, ethylene vinyl acetate-based, polyamide-based, polyolefin-based, polyparaffin-based, epoxy-based, acrylic-based, or other hot melt type adhesive resin can be used. The hot melt adhesive layer in the present invention adheres to a fabric by heating at a temperature preferably in the range of 60° C. to 230° C., more preferably in the range of 80° C. to 210° C., and further preferably in the range of 90° C. to 180° C. The adhering temperature is appropriately selected depending on the heat resistance of a fabric. When the fabric is a common fabric made of chemical fiber, adhering is possible by heating at a temperature in a relatively low temperature range of 150° C. or lower, and when the fabric is made of cotton or heat-resistant fiber, adhering is possible by heating at a temperature of 150° C. or higher, and preferably 180° C or higher.

The thickness of the hot melt adhesive layer is preferably 5 to 200 µm, more preferably 12 to 150 µm, and further preferably 20 to 120 µm.

In the garment-type biological information measurement device, the number of the hot melt adhesive layers may be one or may be two or more.

In the present invention, the stretchable adhesion acceleration layer is provided between the stretchable conductive layer and the stretchable hot melt adhesive layer.

As described above, the adhesion acceleration layer is interposed with respect to the hot melt adhesive which is not always designed in consideration of adhesion to the stretchable conductor composition (stretchable conductive layer). Consequently, the adhesion acceleration layer acts so as to significantly improve adhesion between both layers.

Although the chemical composition of a resin used for the adhesion acceleration layer is not particularly limited, the elongation at break of the adhesion acceleration layer itself is preferably not lower than 50%. In addition, the resin preferably has a crosslinked structure. The resin is required to have no fluidity in a temperature range that is preferably not higher than 60°C, more preferably not higher than 90°C, and further preferably not higher than 120°C.

The resin preferably further has a crosslinked structure. A compound having a low glass transition temperature and having a crosslinked structure can be exemplified by a compound used for a pressure-sensitive adhesive composition, a tackifier, or the like.

As a stretchable resin composition that can be used for the adhesion acceleration layer, it is possible to preferably use a rubber material obtained by introducing a crosslinked structure into urethane rubber, acrylic rubber, silicone rubber, butadiene rubber, rubber containing a nitrile group such as nitrile rubber or hydrogenated nitrile rubber, isoprene rubber, vulcanized rubber, styrene-butadiene rubber, butyl rubber, chlorosulfonated polyethylene rubber, ethylene propylene rubber, vinylidene fluoride copolymer or the like. Further, it is possible to use a mixture of any of these elastomers and a thermosetting resin such as an epoxy resin.

In addition, it is possible to use a resin obtained by introducing a crosslinked structure into a polyurethane resin or a polyester resin having a glass transition temperature that falls within a range of -70°C to 0°C. As the polyurethane resin, it is possible to use a polyurethane elastomer obtained by combining: a soft segment made from a polyether-based polyol, a polyester-based polyol, a polycarbonate-based polyol, or the like; and a hard segment made from diisocyanate or the like. By blending a trifunctional or higher-functional polyol in the polyurethane elastomer, a crosslinked structure can easily be formed. Further, a polyisocyanate compound can also be additionally blended in and reacted with the crosslinked polyurethane elastomer, thereby causing post-crosslinking.

Further, it is possible to use an ultraviolet-curable or electron-beam-curable acrylic resin and also use an ultraviolet-curable (crosslinkable) flexible resin obtained by blending, as necessary, a silane-based coupling agent, a titanate-based coupling agent, an aluminate-based coupling agent, or the like in a blend of a monofunctional (meth)acrylic acid ester and a polyfunctional (meth)acrylic acid ester for imparting a low glass transition temperature after curing.

The flexible resin used for the adhesion acceleration layer in the present invention is preferably a resin that is solidified through a chemical reaction or drying after being applied in liquid form onto the conductive layer having an adhesion surface, in order to achieve strong adhesion to the conductive layer.

Each of an electrode and wiring in the present invention has a layer structure. In the present invention, the electrode refers to an electrode including at least the stretchable conductive layer containing the flexible resin and the conductive filler, the stretchable adhesion acceleration layer, the stretchable hot melt adhesive layer, and a garment fabric in this order. The wiring means that at least a part of the electrode is laminated with an insulation layer. The layer structure is obtained by combining applying, pasting, and lamination as appropriate. At this time, a release film may be temporarily used for convenience of a process.

As the release film used in the present invention, it is possible to use a film obtained by using, as a base material, a polyester-based film of polyethylene terephthalate (PET), polyethylene naphthalate (PEN), polybutylene terephthalate (PBT), or the like and performing release treatment on at least one surface of the base material.

Alternatively, as the release film used in the present invention, it is possible to use a so-called release paper sheet obtained by performing release treatment on one or both surfaces of a paper sheet. That is, although the name "release film" is used in the present invention for convenience, the material of the base material thereof is not particularly limited.

In the release treatment in the present invention, fluororesin coating, silicone resin coating, fluorine plasma treatment, or the like can be exemplified.

Further, in the present invention, it is also possible to use a film or sheet made of a material poor in adhesiveness such as an untreated polyimide film, fluororesin film, silicone resin sheet, polypropylene film, or polyethylene film. Of course, these difficult-to-bond films may be further subjected to a surface treatment.

Further, in the present invention, it is possible to use, as the release film, a metal foil made from a metal called an hard-to-bond metal such as molybdenum, tungsten, chromium, stainless steel, or aluminum. Further, it is possible to use, as the release film, a surface-metalized polymer film obtained by forming any of these metals in the form of a thin film on a surface of a polymer film of PET, PEN, polyimide, or the like through a method such as vacuum deposition or sputtering.

The thickness of the release film of the present invention is preferably 15 to 190 µm, more preferably 24 to 130 µm, and still more preferably 40 to 105 µm. When the thickness of the release film is not within the predetermined range, if only the laminated stretchable conductor sheet is slit, the slit may possibly be short or also cut the release film.

The release film of the present invention may preferably have transparency. The release film of the present invention preferably has an average transmittance for visible light of 30% or more, more preferably 50% or more, and still more preferably 70% or more. If the transparency of the release film is insufficient, positioning when the stretchable conductor sheet in which a slit has been preliminarily formed and the unnecessary portion has been removed is transferred onto a fabric becomes difficult.

The release film of the present invention preferably has certain heat resistance. Heat resistance can be determined by a glass transition temperature or softening temperature. In the present invention, it is preferable that the higher of either the glass transition temperature or the softening temperature be 50° C. or higher, more preferably 65° C. or higher, still more preferably 85° C. or higher, further preferably 125° C. or higher, and still further preferably 175° C. or higher. The heat resistance of the release film is appropriately selected depending on the temperature at which the stretchable conductor sheet adheres to the fabric using the hot melt adhesive layer. In the present invention, the adhesive strength between hot melt adhesive layer and the release film is 0.03 N/cm to 4.0 N/cm, preferably 0.1 N/cm to 2.0 N/cm. When the adhesive strength is below this range, the handling properties of the sheet may deteriorate.

In the present invention, a temporary support body can be used during a process for manufacturing the to-be-pasted-on-garment-fabric conductive laminated sheet. The temporary support body in the present invention is a base on which the stretchable conductive layer and the like are to be formed. The temporary support body is eventually removed.

As the temporary support body, a thing equivalent to the release film can be used. However, the temporary support body needs to have a strength to support the laminated sheet in the middle of manufacturing in a manufacturing process, and thus it is possible to use a PET film, a PEN film, a PP film, a PI film, or the like which are relatively thick and which have thicknesses of about, for example, 100 µm. It is possible to preferably use a temporary support body obtained by performing release treatment on any of these polymer films in order to remove the temporary support body in a subsequent step. Likewise, it is also possible to use a metal plate, a metal foil, a glass plate, or the like obtained by performing release treatment as necessary. In consideration of productivity, the temporary support body is preferably wound into a roll in the middle of a process. Thus, it is recommended to use a long release film.

In the present invention, a stretchable surface insulation layer can be formed as a layer for protecting a surface of the conductive layer. The surface insulation layer can insulate the surface of the conductive layer. A non-thermoplastic film can be used as the surface insulation layer. Further, in the present invention, the non-thermoplastic film can be used as a portion of the to-be-pasted-on-garment-fabric conductive laminated sheet. When the non-thermoplastic film is inserted as an intermediate layer on the hot melt adhesive layer side of the conductive layer, the non-thermoplastic film can be used for the purpose of improving the electric insulation properties, the mechanical strength, the handleability, or the like on the side on which a surface to be adhered on a garment or the like is present.

In principle, a portion at which a cover insulation layer has been formed on the conductive layer can be used as a wiring portion. However, if surface insulation is not particularly needed, the present invention is not limited thereto.

The non-thermoplastic film in the present invention is preferably made from a stretchable resin composition in the same manner as the conductive layer and the hot melt adhesive layer, and a urethane resin, an elastomer having a crosslinked structure or a silicone rubber sheet, or the like can be used. Here, the term "non-thermoplastic" means that liquefaction occurs neither in an environment in which generally used garment fabrics are used nor at a processing temperature used when the garment-type biological information measurement device is manufactured, and a non-thermoplastic film having a high melting point (classified as a thermoplastic resin in a strict sense) may be used as long as the non-thermoplastic film is stretchable at normal temperature. The non-thermoplastic resin film can be preferably pasted on the laminated sheet by using the hot melt adhesive.

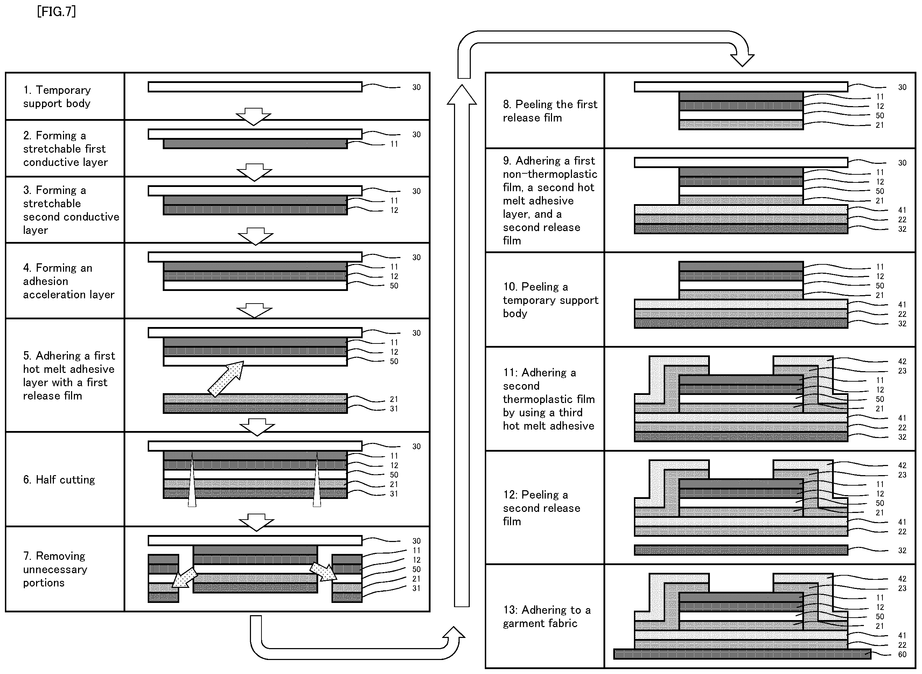

A manufacturing process for an electrode portion and an electrical wiring portion of the garment-type biological information measurement device of the present invention will be described with reference to FIG. 7. The upper and lower sides in each step in FIG. 7 are adjusted according to the upper and lower sides in the final step, and thus the upper and lower sides in step 1 to step 8 in FIG. 7 are opposite to those in an actual operation.

Step 1: First, an example in which a release PET film is used as a temporary support body 30, is considered here. Although a release film can be used as the temporary support body, the temporary support body does not correspond to the release film in the present invention.

Step 2, step 3: A stretchable first conductive layer 11 and a stretchable second conductive layer are formed in this order on the release PET film. Each of the conductive layers can be formed by applying a substance prepared as a solvent-containing paste through an ordinary method and drying the paste. The applying and drying for obtaining the first and second conductive layers may be performed one layer after another. Alternatively, the conductive layers can be formed through a so-called "wet-on-wet" method in which both pastes are applied in double layers and then simultaneously dried. Here, a case where the stretchable conductive layer has a two-layer structure has been described. A conductive layer may be formed by the applying and drying in the same manner also in the case where the conductive layer is formed as a single layer.

Step 4: On the surface of the conductive layer formed on the release PET film in this manner, an adhesion acceleration layer 50 is further formed. As means for forming the adhesion acceleration layer, applying or lamination may be performed according to the material used for the adhesion acceleration layer. As a method for the applying, it is possible to, depending on the material, employ methods such as: a method in which a material in liquid form is applied and dried so as to be solidified; a method in which the material is reacted by means of heating, ultraviolet radiation, or the like so as to be solidified; or a method in which the material is applied through melt-extrusion. As a method for the applying, it is possible to employ a spray coating method, a curtain coating method, a die coating method, a bar coating method, a screen printing method, or the like according to the viscosity of the application liquid.

Step 5: A first hot melt adhesive layer is adhered on the adhesion acceleration layer.

If heat lamination is performed in this step, a first release film can be used so as to be superposed on a surface, of the hot melt adhesive layer, on an opposite side to the adhesion acceleration layer in order to prevent the hot melt adhesive from being adhered on a press heating plate or a heat roll of a laminator.

In this stage, the release PET film as the temporary support body may be removed.

If the first hot melt adhesive layer is positioned on a garment fabric and subj ected to pressure-bonding through heating after the first release film is peeled in this stage, an electrode satisfying the features of the present invention can be obtained, and a minimum configuration of the to-be-pasted-on-garment-fabric conductive laminated sheet in the present invention is obtained. Further, if a stretchable insulating coat is provided on the first conductive layer after the temporary support body is removed, use as electrical wiring is also possible.

For use for an actual garment-type biological information measurement device, an operation of causing the conductive layer and the surface insulation layer to have predetermined shapes is preferably performed. One example of the operation is shown in the next and subsequent steps.

Step 6: The conductive laminated sheet obtained in the preceding step is cut into a predetermined shape. As cutting means, known means such as laser cutting, cutting with a Thomson blade, or press punching may be used. The conductive laminated sheet in the present invention is highly flexible (in plain terms, soft). Thus, if the size of the conductive laminated sheet exceeds a certain value, it becomes difficult to handle the conductive laminated sheet. Therefore, it is possible to use means which is so-called half cutting in which only the conductive laminated sheet portion is cut with the temporary support body being left as is. Half cutting is a useful means particularly in the case where a predetermined pattern has a complicated shape including a thin line. FIG. 7 exemplifies the case where half cutting has been performed.

The temporary support body may be peeled and removed in this stage if no trouble occurs by doing so. If the predetermined shape is a relatively simple shape and a firm material is used for the first release film, the temporary support body can be removed in a relatively early stage.

Step 7: Unnecessary portions resulting from the cutting are removed.

Step 8: The first release film is peeled. The first release film may be peeled immediately after step 5 or immediately after the cutting in step 6.

Step 9: A first non-thermoplastic film, a second hot melt adhesive layer, and a second release film are adhered on the first hot melt adhesive layer. In this step, the second hot melt adhesive layer can be pasted together with the second release film after the first non-thermoplastic film is adhered. Alternatively, a sheet obtained by stacking the first non-thermoplastic film, the second hot melt adhesive layer, and the second release film in advance can also be collectively adhered on the first hot melt adhesive layer.

Step 10: Here, the temporary support body is peeled. As described above, the temporary support body may be peeled before this step.

Step 11: A second non-thermoplastic film is adhered on the entirety or a portion on the conductive layer side. Although the second non-thermoplastic film is adhered by using a third hot melt adhesive in this example, the third hot melt adhesive is unnecessary if the non-thermoplastic film itself has an adhesion function. For example, the second non-thermoplastic film can be formed by applying or laminating a resin in liquid form or in a semi-cured state and then curing the resin through heating, ultraviolet radiation, or the like. Alternatively, the second non-thermoplastic film can be formed by, for example, a method in which the resin is applied in a solution state and dried so as to be solidified. Consequently, a to-be-pasted-on-garment-fabric conductive laminated sheet in which insulated and protected electrical wiring and an electrode for contact with a living body have been combined with each other is obtained.

If the conductive layer in the present invention is used merely as wiring, the conductive layer is preferably coated with the second non-thermoplastic film corresponding to an insulating cover coat layer. Neither a portion, of the conductive layer in the present invention, that serves as an electrode for contact with the surface of a living body, nor a portion thereof that is used for electrical connection to another part such as a connector, is coated with the second non-thermoplastic film. Although not shown in this drawing, a release film may be present on the upper surface (a surface opposite to the adhesion surface) of the second non-thermoplastic film. Further, the release film can also be disposed so as to, instead of covering only the second non-thermoplastic film, cover the entirety including the portions not coated with the second non-thermoplastic film, i.e., the electrode portion for contact with a living body and the portion for electrical connection to another part. The purpose of this is to protect the surface of the electrode and the like when the conductive laminated sheet is adhered on a garment fabric by using a pressing machine or the like.

The timing of peeling the release film is as follows. Basically, the release film only has to be peeled immediately before certain processing is performed on the surface on which the release film is pasted. Meanwhile, if the release film needs to be peeled for convenience of handling or processing such as cutting, the release film may be peeled as necessary at the appropriate timing. In addition, after the release film is peeled once, covering with a release film, a protective film, or the like may be performed again.

Step 12: The second release film is peeled.

Step 13: Superposition is performed with an orientation taken such that the second hot melt adhesive layer is brought into contact with a garment fabric, and pressure-bonding through heating is performed, whereby a garment having the electrode and the electrical wiring is obtained.

### EXAMPLES

Hereinafter, the present invention will be more specifically described by means of examples. However, the present invention is not limited by the following examples and can also be carried out with modifications being made within the scope of the gist described above and below, and each of these modifications are included in the technical scope of the present invention.

A conductive silver paste, a conductive carbon paste, and an adhesion acceleration layer forming resin used in the following examples were prepared as follows.

### [Preparation Of Stretchable Conductive Silver Paste (Second Stretchable Conductor Composition)]

20 parts by mass of a nitrile rubber (NBR) [Nipol (registered trademark) "DN003" manufactured by Zeon Corporation] as a flexible resin (binder) was dissolved in 80 parts by mass of isophorone, whereby an NBR solution was made. 110 parts by mass of a silver powder (an aggregated silver powder "G-3 5" manufactured by DOWAElectronics Materials Co., Ltd., and having an average particle diameter of 5.9 µm) was blended in 100 parts by mass of the obtained NBR solution, and the mixture was kneaded by using a three-roll mill, whereby a conductive silver paste AG was produced.

A fundamental evaluation of the obtained conductive silver paste was performed as follows. That is, screen printing with the paste was performed such that the thickness thereof was 25 µm, and the paste was dried at 100°C for 20 minutes, whereby a stretchable conductive layer (stretchable silver conductor sheet) was obtained. It was confirmed that: the stretchable silver conductor sheet had an initial specific resistance of 250 µΩ·cm; and the stretchable silver conductor sheet had stretchability and was stretchable so as to maintain conductivity thereof even after 20%-stretching was repeated 100 times.

### [Preparation Of Stretchable Conductive Carbon Paste (First Stretchable Conductor Composition)]

A nitrile rubber [Nipol (registered trademark) "DN003" manufactured by Zeon Corporation] as a flexible resin binder, 20 parts by mass of KETJENBLACK (registered trademark) EC300J manufactured by Lion Specialty Chemicals Co., Ltd., and 50 parts by mass of ethylene glycol monoethyl ether acetate as a solvent, were preliminarily stirred and then dispersed by using a three-roll mill, whereby a conductive carbon paste CB was obtained.

A fundamental evaluation of the obtained conductive carbon paste was performed as follows. That is, screen printing with the paste was performed such that the thickness thereof was 25 µm, and the paste was dried at 100°C for 20 minutes, whereby a stretchable conductive layer (stretchable carbon conductor sheet) was obtained. It was confirmed that: the stretchable carbon conductor sheet had an initial specific resistance of 0.14 Ω·cm; and the stretchable carbon conductor sheet had stretchability and was stretchable so as to maintain conductivity thereof even after 20%-stretching was repeated 100 times.

### [Stretchable Adhesion Acceleration Layer Forming Resin AD1]

18 parts by mass of a nitrile rubber (NBR) [Nipol (registered trademark) "DN003" manufactured by Zeon Corporation] and 1.4 parts by mass of a bisphenol A type epoxy resin (jER (registered trademark) 1001) manufactured by Mitsubishi Chemical Corporation were dissolved in 80 parts by mass of isophorone, whereby an NBR-epoxy mixed solution was obtained. 0.6 parts by mass of an epoxy curing agent (YN100) manufactured by Mitsubishi Chemical Corporation was further added to the obtained NBR-epoxy mixed solution, and stirring and mixing were performed, whereby an adhesion acceleration layer forming application liquid AD1 was obtained.

### [Stretchable Adhesion Acceleration Layer Forming Resin AD2]

2 parts by mass of 3-methacryloxypropylmethyldimethoxysilane "KBM-502" manufactured by Shin-Etsu Chemical Co., Ltd., and 2 parts by mass of a titanate-based coupling agent "PLENACT (registered trademark) 238S" manufactured by Ajinomoto Fine-Techno Co., Inc., were added to 96 parts by mass of a one-component UV-curable elastic adhesive "SX-UV220" which contained an acrylic copolymer as a main component and which was manufactured by CEMEDINE Co., Ltd., and stirring and mixing were performed, whereby a UV-curable adhesion acceleration layer forming application liquid AD2 was obtained.

### [Stretchable Adhesion Acceleration Layer Forming Resin AD3]

20 parts by mass of a polyester resin "VYLON (registered trademark) 550" (Tg=-15°C, hydroxyl value: 4 mg/KOH) manufactured by Toyobo Co., Ltd., 70 parts by mass of a polyester urethane resin "VYLON UR8700" (Tg=-22°C, hydroxyl value: 4 mg/KOH) manufactured by Toyobo Co., Ltd., and 10 parts by mass of an aromatic polyisocyanate curing agent "CORONATE (registered trademark) L" manufactured by Nippon Polyurethane Industry Co., Ltd., were dissolved in 200 parts by mass of a solvent mixture (a mass ratio of methyl ethyl ketone/toluene/cyclohexanone=1/1/1), whereby an adhesion acceleration layer forming application liquid AD3 was obtained.

### (Example 1)

A release PET film having a surface treated with a silicone-based release agent was used as a temporary support body, and, according to the process shown in FIG. 7, the stretchable conductive carbon paste CB was applied on the release layer side of the release PET film by using a bar coater so as to have a post-drying thickness of 15 µm and was dried. Consequently, a first conductive layer was obtained.

Then, the stretchable conductive silver paste AG was applied on the first conductive layer by using a bar coater so as to have a post-drying thickness of 35 µm and was dried. Consequently, a second conductive layer was obtained.

Then, the adhesion acceleration layer forming resin AD1 was applied on the second conductive layer by using a bar coater so as to have a post-drying film thickness of 5 µm and was heated at 100°C for 30 minutes. Consequently, the epoxy compound was reacted simultaneously with drying, whereby an adhesion acceleration layer was formed.

Then, as a first hot melt adhesive layer/first release film, a hot melt urethane sheet MOBILON (registered trademark) MOB100 (polyurethane hot melt sheet/release paper sheet) manufactured by Nisshinbo Holdings Inc., was superposed on the adhesion acceleration layer, with an orientation taken such that the hot melt sheet side of the MOB 100 was brought into contact with the adhesion acceleration layer. The MOB100 was pressed and heated so as to be adhered on the adhesion acceleration layer by using a hot-pressing machine under a condition of a pressure of 0.5 kg/cm², a temperature of 130°C, and a pressing time of 20 seconds. Further, the temporary support body was peeled, whereby a to-be-pasted-on-garment-fabric conductive laminated sheet (S1) was obtained.

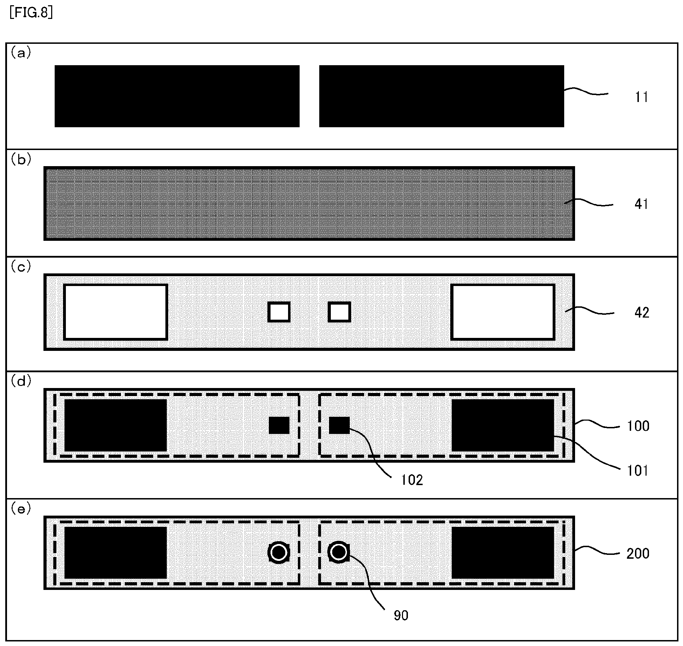

The obtained to-be-pasted-on-garment-fabric conductive laminated sheet was punched into a shape in FIG. 8(a) by using a Thomson blade. Next, the release paper sheet was peeled, and then the first hot melt adhesive layer was superposed so as to be in contact with a chest portion on the reverse side of a T-shirt having a medium size and made from a double tricot fabric resulting from mixed spinning of 70% of polyester and 30% of cotton, the chest portion being a portion to be located on the inner side of the T- shirt and to be brought into contact with the human body at the time of normal wearing. The to-be-pasted-on-garment-fabric conductive laminated sheet was pressed and heated so as to be pasted on the chest portion by using a hot-pressing machine under the condition of a pressure of 0.5 kg/cm², a temperature of 130°C, and a pressing time of 20 seconds.

Then, MOBILON (registered trademark) MF-10F3 (non-thermoplastic polyurethane sheet/polyurethane hot melt sheet/release paper sheet) was punched into a shape in FIG. 8(c) by using a Thomson blade. The release paper sheet was peeled. The MF-10F3 was superposed with an orientation taken such that the hot melt adhesive layer thereof was brought into contact with the conductive layer. The superposition was performed such that: edges of the conductive layer were coated with the MF-10F3 as in FIG. 8(d); and the conductive layer was exposed at electrode portions thereof and portions thereof to be connected to connectors. The MF-10F3 was pressed and heated so as to be pasted on the conductive layer by using a hot-pressing machine under the condition of a pressure of 0.5 kg/cm², a temperature of 130°C, and a pressing time of 20 seconds.

Then, as shown in FIG. 8(e), male parts of snap fasteners, as connectors, made of stainless steel were attached such that studs thereof faced a side opposite to the skin side. The snap fasteners ensure electrical contact with the conductive layer through riveting performed by using holes formed so as to penetrate the conductive laminated sheet and the T-shirt fabric. After the above process, a heart rate sensor "WHS-3" manufactured by UNION TOOL Co. was connected to the connectors, whereby a T-shirt capable of measuring an electrocardiographic waveform, i.e., a garment-type biological information measurement device (WT1), was obtained.

### (Example 2)

Operations up to the step of forming the second conductive layer were the same as those for the sheet S1. Then, the adhesion acceleration layer forming resin AD2 was applied on the second conductive layer by using a bar coater so as to have a post-curing film thickness of 10 µm, and was irradiated with ultraviolet light by using a high pressure mercury lamp such that the accumulated light amount thereof was 1000 mJ/cm². Consequently, an adhesion acceleration layer was formed. From this step on, the hot melt adhesive sheet/release film was adhered in the same manner as that for the sheet S 1, whereby a to-be-pasted-on-garment-fabric conductive laminated sheet (S2) was obtained. The process is further advanced in the same manner, whereby a garment-type biological information measurement device (WT2) was obtained.

### (Example 3)

Operations up to the step of forming the second conductive layer were the same as those for the sheet S1. Then, the adhesion acceleration layer forming resin AD3 was applied on the second conductive layer by using a bar coater so as to have a dry film thickness of 3 µm, and a crosslinking reaction was made to keep occurring simultaneously with drying at 100°C for 30 minutes, whereby an adhesion acceleration layer was formed. From this step on, the hot melt adhesive sheet/release film was adhered in the same manner as that for the sheet S1, whereby a to-be-pasted-on-garment-fabric conductive laminated sheet (S3) was obtained. The process is further advanced in the same manner, whereby a garment-type biological information measurement device (WT3) was obtained.

### (Example 4)

Operations up to the step of forming the first conductive layer were the same as those for the sheet S1. Then, the adhesion acceleration layer forming resin AD1 was applied on the first conductive layer by using a bar coater so as to have a dry film thickness of 12 µm, and a crosslinking reaction was made to keep occurring simultaneously with drying at 100°C for 30 minutes, whereby an adhesion acceleration layer was formed. From this step on, the hot melt adhesive sheet/release film was adhered in the same manner as that for the sheet S1, whereby a to-be-pasted-on-garment-fabric conductive laminated sheet (S4) was obtained. The process is further advanced in the same manner, whereby a garment-type biological information measurement device (WT4) was obtained.

### (Comparative Example 1)

Operations up to the step of forming the second conductive layer were the same as those for the sheet S1. Meanwhile, the step of forming an adhesion acceleration layer was not performed, and from this step on, the hot melt adhesive sheet/release film was adhered in the same manner as that for the sheet S1, whereby a to-be-pasted-on-garment-fabric conductive laminated sheet (S0) was obtained. The process is further advanced in the same manner, whereby a garment-type biological information measurement device (WT0) was obtained.

Each of three healthy males respectively in their twenties, thirties, and forties was asked to wear each of the obtained garment-type biological information measurement devices. For each of the males, an electrocardiographic waveform at rest, and an electrocardiographic waveform as one during exercise obtained from the male performing radio calisthenics No. 1 and radio calisthenics No. 2, were observed. The electrocardiographic waveforms from each of the males were able to be measured without any problems.

### (Evaluation Of Durability Against Washing)

The initial resistance value between each electrode portion and the corresponding connector connection portion of each of the obtained garment-type biological information devices was measured. The results are indicated in Table 1. In the case of a resistance value of not higher than 100 Q, the resistance value was measured through Kelvin connection by using Milliohmmeter 4338B manufactured by Agilent Technologies, Inc. In the case of a resistance value of higher than 100 Q, the resistance value was measured by using a digital tester.

Then, each sample to be evaluated for durability against washing was washed by using a household washing machine (ASW-50F5 (HS) manufactured by SANYO Electric Co., Ltd.) and a washing test automatic control device (Tsujii Senki Kogyo Co., Ltd., type SAD-135) with a wash net on the basis of a 5-fold acceleration test (dried in shade one time after 5 cycles of washing in succession) according to the test method specified in No.103 in Testing methods for woven and knitted fabrics of JIS L 1096. This washing was performed a total of 100 cycles.

The water flow was "strong", the bath ratio was 1:30, the washing water temperature was 40°C, the washing time was 5 minutes, the rinsing water temperature was 25°C, the rinsing time was 2 minutes, and a powder type of "Attack" was used as a detergent. Here, 1 cycle (one time) of washing is a cycle that involves stirring, dehydration, rinsing, dehydration, rinsing, and then dehydration inside a detergent aqueous solution in a washing tank. Necessary amounts of dummy T-shirts were used in order to satisfy the bath ratio. The resistance value was measured after drying in shade at the elapse of every 5 cycles of washing.

Resistance values obtained after 100 cycles of washing are indicated in Table 1. In the case where resistance measurement became unable to be performed owing to disconnection in the middle of the test, the fact that the disconnection had occurred was recorded.

After 100 times of washing, each of the three males was asked to wear each garment-type biological information measurement device and underwent an operation test in the same manner as in the initial state. In each of Examples 1 to 4, electrocardiographic measurement was able to be performed without any problem. Meanwhile, in Comparative Example 1 in which disconnection occurred, electrocardiographic measurement was unable to be performed as a matter of course.

### (Example 5)

Operations up to the step that preceded peeling of the temporary support body were performed in the same manner as in Example 1, whereby a temporary-support-body-attached conductive laminated sheet S1 was obtained. Half cutting was performed with a Thomson blade from the release film side as shown in step 6 in FIG. 7, and unnecessary portions were removed as in step 7, whereby a predetermined electrode-and-wiring pattern shown in FIG. 8(a) was obtained. Further, the first release film was peeled as in step 8.

A hot melt urethane sheet MOBILON (registered trademark) MF-10F3 (non-thermoplastic polyurethane sheet/polyurethane hot melt sheet/release paper sheet) manufactured by Nisshinbo Holdings Inc., was cut into a predetermined base insulation layer pattern shown in FIG. 8(b). On a surface, of the conductive laminated sheet, from which the first release film had been peeled, the MF-10F3 was superposed with an orientation taken such that the non-thermoplastic polyurethane sheet thereof (corresponding to the first non-thermoplastic film) was brought into contact with the surface. Then, the MF-10F3 was pressed and heated so as to be adhered on the surface by using a hot-pressing machine under the condition of a pressure of 0.5 kg/cm², a temperature of 130°C, and a pressing time of 20 seconds (step 9).

Then, the temporary support body was peeled (step 10). MOBILON (registered trademark) MF-10F3 having been cut into a predetermined cover insulation layer pattern shown in FIG. 8(c) was superposed such that the hot melt adhesive layer side thereof was brought into contact with the first conductive layer, the MF-10F3 was pressed so as to be adhered on the first conductive layer in the same manner, and then the release paper sheet annexed to the MF-10F3 was peeled (step 11). Consequently, a conductive laminated sheet S10 (FIG. 8(d)) including the base insulation layer and the cover insulation layer was obtained. Here, portions of the conductive layer that are exposed from large window portions present in the cover insulation layer pattern correspond to electrode portions, portions of the conductive layer that are exposed from small window portions present in the cover insulation layer pattern correspond to connection portions to which connectors are attached, and portions each located between the corresponding electrode portion and the corresponding connection portion and coated with the cover insulation layer correspond to wiring portions.

### (Examples 6 to 8 and Comparative Example 2)

The same additional processing as that in Example 5 was performed on the temporary-support-body-attached conductive laminated sheets S2, S3, and S3 obtained in Examples 2 to 4, whereby conductive laminated sheets S20, S30, and S40 were respectively obtained. The same additional processing as that in Comparative Example 1 was also performed, whereby a conductive laminated sheet S00 was obtained for Comparative Example 2.

Each of the obtained conductive laminated sheets was pressed and heated so as to be adhered on the skin side of an underband of a brassiere by using a hot-pressing machine under a condition of a pressure of 0.5 kg/cm², a temperature of 130°C, and a pressing time of 40 seconds. Then, as shown in FIG. 8(e), male parts of snap fasteners, as connectors, made of stainless steel were attached such that studs thereof faced a side opposite to the skin side. The snap fasteners ensure electrical contact with the conductive layer through riveting performed by using holes formed so as to penetrate the conductive laminated sheet and an underband fabric of the brassiere. A heart rate sensor "WHS-3" manufactured by UNION TOOL Co. was connected to the connectors, whereby a brassiere capable of measuring an electrocardiographic waveform, i.e., a garment-type biological information measurement device, was obtained.

Each of three healthy females respectively in their twenties, thirties, and forties was asked to wear each of the obtained garment-type biological information measurement devices. For each of the females, an electrocardiographic waveform at rest, and an electrocardiographic waveform as one during exercise obtained from the female performing radio calisthenics No. 1 and radio calisthenics No. 2, were observed. The electrocardiographic waveforms from each of the females were able to be measured without any problems. Then, 100 cycles of washing tests were performed under the same conditions as those in Example 1, and electrocardiographic waveform measurement was performed with the garment-type biological information measurement devices being worn in the same manner. The results are indicated in Table 2. In the case of using the conductive laminated sheets in Examples 5 to 8, electrocardiographic waveform measurement was able to be performed in the same manner as in the initial state. Meanwhile, in Comparative Example 2, electrocardiographic waveforms from the wearers at rest were able to be observed, but, during exercise, noises were mixed in waveforms so that no well-shaped waveforms were able to be observed. After the operation checks, the conductivity between each snap fastener portion and the corresponding electrode portion of the conductive laminated sheet pasted on each brassiere was checked. As a result of the check, although conductivity was ensured in both the examples and the comparative examples, peeling and fall-off of the conductive layer were observed at a portion of the electrode portion in Comparative Example 2.

**[Table1]**

| | | Comparative Example 1 | Example 1 | Example 2 | Example 3 | Example 4 |
|---|---|---|---|---|---|---|
| Garment-type biological information measurement device | | WT0 | WT1 | WT2 | WT3 | WT4 |
| Conductive laminated sheet | | S0 | S1 | S2 | S3 | S4 |
| First conductive layer | | CB (15 *µ*m) | CB (15 *µ*m) | CB (15 *µ*m) | CB (15 *µ*m) | CB (50 *µ*m) |
| Second conductive layer | | AG (35 *µ*m) | AG (35 *µ*m) | AG (35 *µ*m) | AG (35 *µ*m) | No |
| Adhesion acceleration layer | | No | AD1 (5 *µ*m) | AD2 (10 *µ*m) | AD3 (3 *µ*m) | AD1 (12 *µ*m) |
| First hot melt adhesive layer | | MOB100 | MOB100 | MOB100 | MOB100 | MOB100 |
| First release film | | | | | | |
| Initial operation check | | Good | Good | Good | Good | Good |
| Operation check after washing test | | Bad | Good | Good | Good | Good |
| Initial wiring resistance | [Ω] | 60 | 57 | 63 | 54 | 25k |
| Wiring resistance after washing test | [Ω] | Disconnection after 50 times of washing | 240 | 260 | 19k | 48k |

**[Table2]**

| | | Comparative Example 2 | Example 5 | Example 6 | Example 7 | Example 8 |
|---|---|---|---|---|---|---|
| Conductive laminated sheet | | S00 | S10 | S20 | S30 | S40 |
| First conductive layer | | CB (15 *µ*m) | CB (15 *µ*m) | CB (15 *µ*m) | CB (15 *µ*m) | CB (50 *µ*m) |
| Second conductive layer | | AG (35 *µ*m) | AG (35 *µ*m) | AG (35 *µ*m) | AG (35 *µ*m) | No |
| Adhesion acceleration layer | | No | AD1 (5 *µ*m) | AD2 (10 *µ*m) | AD3 (3 *µ*m) | AD1 (12 *µ*m) |
| First hot melt adhesive layer | | HM layer of MOB100 | HM layer of MOB100 | HM layer of MOB100 | HM layer of MOB100 | HM layer of MCB100 |
| First non-thermoplastic film | | MF-10F3 | MF-10F3 | MF-10F3 | MF-10F3 | MF-10F3 |
| Second hot melt adhesive layer | | | | | | |
| Second non-thermoplastic film | | MF-10F3 | MF-10F3 | MF-10F3 | MF-10F3 | MF-10F3 |
| Third hot melt adhesive layer | | | | | | |
| Initial operation test | Resting | Good | Good | Good | Good | Good |
| | During exercise | Good | Good | Good | Good | Good |
| Operation test after washing test | Resting | Good | Good | Good | Good | Good |
| | During exercise | Noises were mixed | Good | Good | Good | Good |

### INDUSTRIAL APPLICABILITY

As described above, since the biological information measurement device of the present invention is provided with the adhesion acceleration layer between the stretchable conductive layer and the hot melt adhesive layer, the biological information measurement device can realize an electrode and wiring, for biological information measurement, having an excellent durability against washing in a state of being pasted on a garment fabric.

Although electrocardiographic measurement has been described in EXAMPLES by taking, as examples, T-shirts and brassieres for females, garments are not limited to those for males, those for females, those for the upper half of the body, and those for the lower half of the body, and the present invention is applicable to electrode-equipped biological information measurement garments over a wide range. Specifically, the present invention is applicable to protective garments for use in various kinds of sports, martial arts, work sites, safeguarding, and the like. In addition, the present invention is applicable to garments for use in hospitals and garments for use in caregiving that are, in many cases, taken on or off by caregivers or medical staff when the wearers are in bed. As a matter of course, the present invention is applicable not only to electrocardiographic measurement but also to myopotential measurement and brain wave measurement. In addition, the present invention is applicable, just as electrical wiring, to part elements such as various sensors, power supplies, antennas, and coils. Further, the present invention is usable for electrode portions and wiring portions of, for example, devices for electrical muscle stimulation, thermomassage, and electromagnetic treatment.

### DESCRIPTION OF REFERENCE SIGNS

1 : stretchable conductive layer
2 : hot melt adhesive layer
4 : cover insulation layer
5 : adhesion acceleration layer
11 : stretchable first conductive layer
12 : stretchable second conductive layer
21 : first hot melt adhesive layer
22 : second hot melt adhesive layer
23 : third hot melt adhesive layer
30 : temporary support body
31 : first release film
32 : second release film
41 : first non-thermoplastic film
42 : second non-thermoplastic film
50 : adhesion acceleration layer
60 : garment fabric
90 : snap fastener (connector)
100 : conductive laminated sheet including a base insulation and a cover insulation
101 : electrode portion
102 : connection portion
200 : conductive laminated sheet including a connector, a base insulation and the cover insulation

## Claims

1. A garment-type biological information measurement device comprising
an electrode, wherein
the electrode comprises
a stretchable conductive layer containing a flexible resin and a conductive filler,
a stretchable adhesion acceleration layer,
a stretchable hot melt adhesive layer, and
a garment fabric, in this order.

2. The garment-type biological information measurement device according to claim 1, wherein
the device further comprises a stretchable surface insulation layer disposed on a side, of the conductive layer, that is brought into contact with a living body.

3. The garment-type biological information measurement device according to claim 1 or 2, wherein
the conductive layer has a structure with two or more layers including
a stretchable first conductive layer containing at least a flexible resin and a carbon-based conductive filler as constituent components, and
a stretchable second conductive layer containing at least a flexible resin and a metal-based conductive filler as constituent components.

4. A manufacturing method for the garment-type biological information measurement device according to claim 1 or 2,
the manufacturing method comprising:
making, on a temporary support body, a to-be-pasted-on-garment-fabric conductive laminated sheet through a process comprising at least
a step of forming a stretchable conductive layer containing a flexible resin and a conductive filler,
a step of forming a stretchable adhesion acceleration layer on the conductive layer, and
a step of forming a stretchable hot melt adhesive layer on the adhesion acceleration layer; and
pasting the obtained to-be-pasted-on-garment-fabric conductive laminated sheet on a garment via the hot melt adhesive layer, to form an electrode on the garment.

5. A manufacturing method for the garment-type biological information measurement device according to claim 3,
the manufacturing method comprising:
making, on a temporary support body, a to-be-pasted-on-garment-fabric conductive laminated sheet through a process comprising at least
a step of forming a stretchable first conductive layer containing at least a flexible resin and a carbon-based conductive filler as constituent components,
a step of further forming a stretchable second conductive layer containing at least a flexible resin and a carbon-based conductive filler as constituent components,
a step of forming a stretchable adhesion acceleration layer on the second conductive layer, and
a step of forming a stretchable hot melt adhesive layer on the adhesion acceleration layer; and
pasting the obtained to-be-pasted-on-garment-fabric conductive laminated sheet on a garment via the hot melt adhesive layer, to form an electrode on the garment.

6. The garment-type biological information measurement device according to any one of claims 1 to 3, wherein
the adhesion acceleration layer contains an elastomer having a crosslinked structure.

7. The garment-type biological information measurement device according to any one of claims 1 to 3, wherein
the adhesion acceleration layer contains a urethane resin having a crosslinked structure.

8. The garment-type biological information measurement device according to any one of claims 1 to 3, wherein
the adhesion acceleration layer contains an active energy ray-curable acrylic resin composition.

9. The manufacturing method for the garment-type biological information measurement device according to claim 4 or 5, wherein
the adhesion acceleration layer contains an elastomer having a crosslinked structure.

10. The manufacturing method for the garment-type biological information measurement device according to claim 4 or 5, wherein
the adhesion acceleration layer contains a urethane resin having a crosslinked structure.

11. The manufacturing method for the garment-type biological information measurement device according to claim 4 or 5, wherein
the adhesion acceleration layer contains an active energy ray-curable acrylic resin composition.
